# DEMANDE DE BREVET EUROPEEN

(11) **EP 2 813 181 A1**
(43) Date de publication de la demande: **17.12.2014**
(21) Numéro de dépôt: 13290134.9
(22) Date de dépôt: 12.06.2013
(51) Int. Cl.: A61B 5/15, A61J 1/06, A61J 1/10

(54) **Dispositif de prélèvement d'une petite quantité de sang contenue dans une tubulure fermée d'une poche de transfusion sanguine.**

(71) Demandeur: Chabrier Rey, Catherine, 74160 Feigeres (FR)
(72) Inventeur: Chabrier Rey, Catherine, 74160 Feigeres (FR)
(74) Mandataire: Micheli & Cie SA

(57) **Abrégé**

Le dispositif de prélèvement d'une petite quantité de sang contenu dans la tubulure fermée (1) d'une poche de transfusion sanguine, notamment pour réaliser un Contrôle Pré-transfusionnel Ultime (CPU), comprend une pipette (7, 8) reliée à ladite tubulure fermée (1). Il se distingue par une membrane à valve anti-reflux intégrée dans la paroi de la tubulure fermée au point de liaison de celle-ci avec la pipette (7, 8); et qu'il comporte encore une enveloppe protectrice (9), moulée de façon étanche sur la tubulure fermée (1) autour de la pipette (8) et de son petit conduit (7). L'enveloppe de protection s'ouvre par écartement de ses parois et reste sur la tubulure afin de permettre la préhension de la seule pipette. Le tout est agencé de manière à ce que la pipette puisse être séparée manuellement de la tubulure fermée (1) par un mouvement de torsion.

## Description

Une poche de transfusion sanguine comporte généralement une tubulure fermée qui, lorsque la poche de transfusion est pleine de sang, est également remplie du liquide à transfuser. Cette tubulure fermée peut ensuite être divisée par soudure en plusieurs sections contenant chacune une quantité de sang. Lorsqu'il faut effectuer un contrôle ou une vérification du contenu de la poche de transfusion il est ainsi possible de prélever, par sectionnement de l'extrémité de la tubulure fermée, une quantité de sang pouvant être analysée.

Pour prélever, de la tubulure fermée, le sang nécessaire lors du contrôle Pré-transfusionnel ultime (CPU) obligatoire, effectué au lit du patient, avant toute transfusion sanguine, le procédé actuel, oblige la section de cette tubulure avec une paire de ciseaux ce qui engendre une souillure avec les risques de contamination qui peuvent en découler, en outre la grosseur des gouttes ainsi obtenues n'est pas le garant de précision.

La titulaire a développé un dispositif de prélèvement du contenu de la tubulure fermée d'une poche de transfusion décrit dans le document FR 2 971 694 qui permet de limiter les risques de contamination lors du Contrôle Pré-transfusionnel Ultime (CPU) qui comporte une pipette reliée à ladite tubulure fermée de la poche de transfusion qui peut être remplie du contenu de cette tubulure et séparée de celle-ci pour pouvoir ensuite déposer des gouttes de sang sur un carton pré-infusionnel comportant les réactifs nécessaires à la détermination du groupe sanguin et ainsi voir si le contenu de la poche de transfusion est compatible avec le sang du patient receveur. Ce dispositif limite les risques de contamination lors du CPU mais fragilise la tubulure fermée de la poche de transfusion, ce qui impose de prendre des précautions lors de sa manipulation notamment lorsque cette poche de transfusion est centrifugée pour éviter une séparation intempestive entre la pipette et ladite tubulure fermée de la poche de transfusion qui entraînerait une contamination de la centrifugeuse.

La présente invention a pour objet un dispositif de prélèvement d'une petite quantité de sang d'une tubulure fermée d'une poche de transfusion sanguine évitant tous risques de séparation intempestive de la pipette lors de la manutention de la poche de transfusion et de sa centrifugation.

Ce dispositif de prélèvement d'une petite quantité de sang d'une tubulure fermée d'une poche de transfusion obviant aux inconvénients précités se distingue par les caractéristiques énoncées à la revendication 1.

L'invention a également pour objet une poche de transfusion dont la tubulure fermée comporte un dispositif de prélèvement selon la revendication 1.

Le dessin annexé illustre schématiquement et à titre d'exemple une poche de transfusion sanguine et son dispositif de prélèvement selon l'invention.
La figure 1 illustre une poche de transfusion avec sa tubulure fermée munie du dispositif de prélèvement.
La figure 2 illustre la poche de transfusion dans une centrifugeuse.

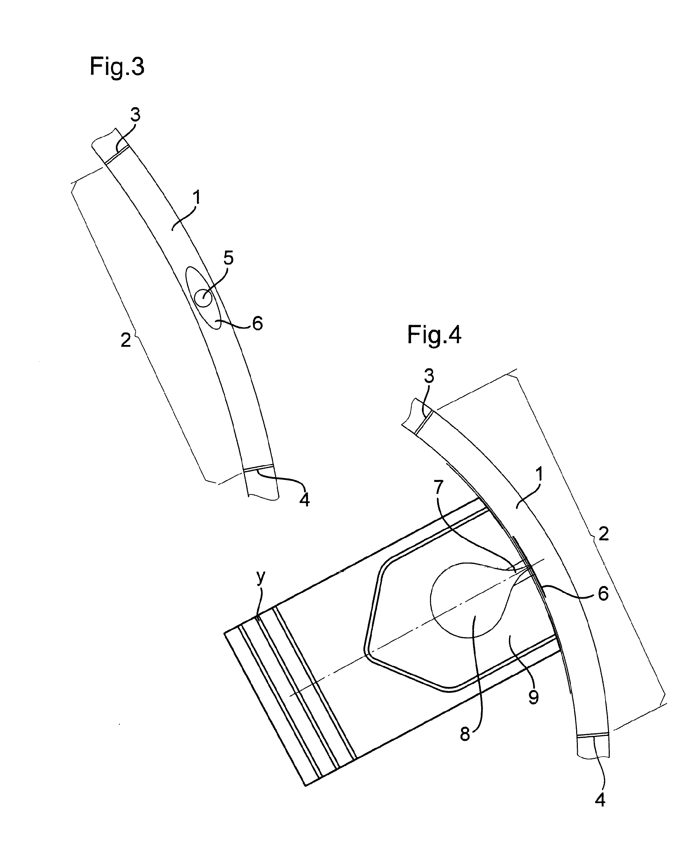

La figure 3 illustre une portion de la tubulure fermée munie d'une membrane à valve anti-retour ou auto-seal.
La figure 4 illustre une portion de la tubulure fermée munie d'une pipette de prélèvement et de son enveloppe de protection.

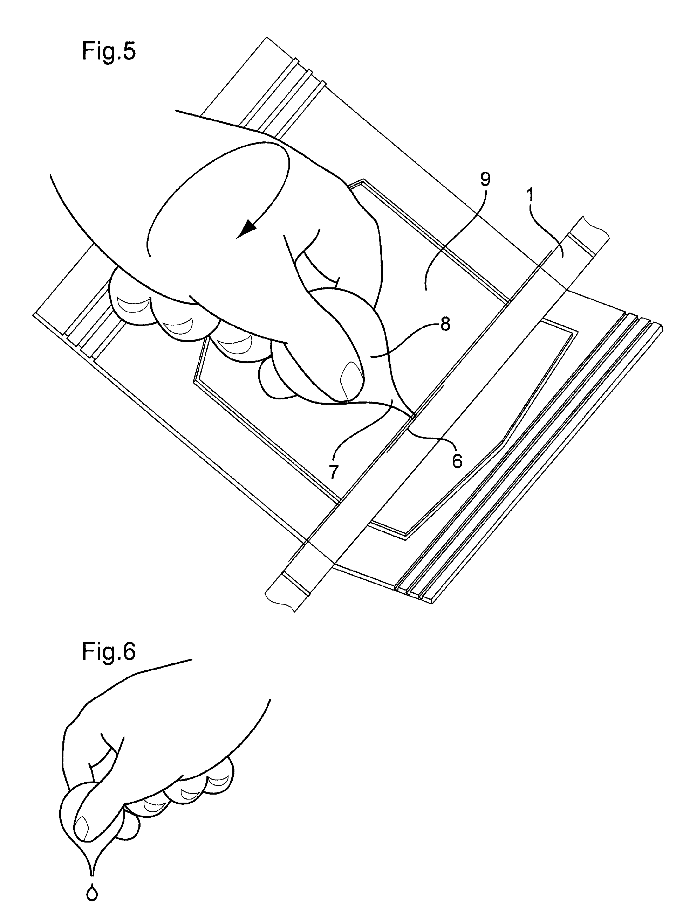

La figure 5 illustre le remplissage de la pipette par l'usager puis sa séparation d'avec la tubulure fermée.
La figure 6 illustre l'expulsion d'une goutte de sang de la pipette par l'utilisateur.

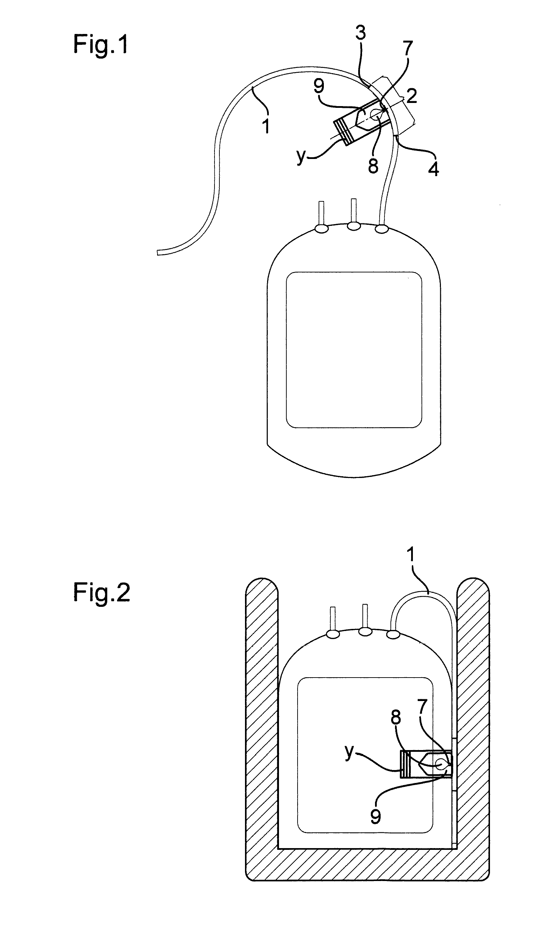

La poche de transfusion illustrée à la figure 1 comporte une tubulure fermée 1 servant à des prélèvements de faible quantité du liquide contenu dans la poche de transfusion pour des besoins d'analyses. Cette tubulure fermée 1 comporte une section 2 voisine de sa connexion à la poche isolée par deux soudures 3, 4 dont le volume est supérieur à ce qui est nécessaire pour pratiquer un Contrôle Pré-transfusionnel Ultime (CPU).

Cette section 2 ou tronçon de la tubulure fermée 1 est munie du dispositif de prélèvement d'une petite quantité du même liquide que celui contenu dans la poche de transfusion et comporte un orifice 5 pratiqué dans la paroi de la tubulure fermée 1 obturé par une membrane 6 composée d'une valve anti-reflux qui lors du retrait de la pipette se referme et redevient étanche dite membrane auto-seal ou auto réparatrice.

La pipette en forme de ballonnet 8 terminée par un petit conduit 7 se fixe de façon étanche sur la paroi de la tubulure fermée 1 au point d'orifice 5. La contenance de cette pipette 8 est de quelques gouttes, généralement plus ou moins vingt gouttes, soit une valeur de l'ordre de 1 ml. Cette quantité de liquide pouvant être aspirée dans la pipette 8 est suffisante pour effectuer un contrôle Pré-transfusionnel Ultime (CPU) mais reste faible pour éviter tous risques de contamination. Le petit conduit 7 présente un diamètre ou section droite inférieure à celle de la tubulure fermée, par exemple comprise entre 0,1 à 0.5 fois ladite section de la tubulure fermée 1. En outre, cette pipette avec petit conduit 7 est fixée sur, ou venue d'une pièce de fabrication avec la paroi de la tubulure fermée 1 de manière à pouvoir en être séparée par simple torsion.

Enfin le dispositif de prélèvement comporte encore une enveloppe protectrice 9, soudée sur la tubulure. Cette protection est fabriquée en usine dans le même matériau que la poche et la tubulure, sa taille de petite dimension insère ou entoure la pipette et la protège pendant la centrifugation. L'élément de protection doit pouvoir s'ouvrir et rester sur la tubulure afin de permettre la préhension pour son remplissage et le contrôle CPU de la seule pipette. A cet effet cet élément de protection est, par exemple, constitué de deux feuilles soudées ou venues de fabrication avec la tubulure fermée par l'une de leurs extrémités. Ces feuilles comportent des formations d'emboîtement permettant de les relier de façon étanche autour de la pipette. Cette enveloppe protectrice s'ouvre par écartement de ses parois pour permettre la préhension de la pipette mais reste solidaire de la tubulure fermée.

Cette enveloppe protectrice 9 est suffisamment souple pour permettre les manipulations de la tubulure fermée 1 sans être endommagée.

Enfin, l'enveloppe protectrice 9 est petite de façon à ce que l'ensemble poche, tubulure, pipette soit d'une maniabilité à toutes épreuves, sans risquer de devenir une gêne lors des différentes étapes que sont:
a) l'emballage individuel de chaque poche en usine,
b) le prélèvement du sang,
c) le conditionnement au laboratoire et la centrifugation,
d) le stockage en chambre froide,
e) et la préparation auprès du patient.

Le prélèvement d'une petite quantité de sang s'effectue de la façon suivante avec le dispositif de prélèvement selon l'invention:

Lors du contrôle le manipulateur ouvre facilement, par le haut cette protection, en tirant de chaque côté des bords dont la fabrication est prévue à cet effet pour séparer les deux feuilles et donner ainsi accès à la pipette.

La pipette 8 ainsi libérée peut être saisie seule, l'usager comprime la pipette, puis relâche sa pression, cette manoeuvre chassant l'air contenu dans la pipette provocant ainsi l'aspiration de la quantité de sang voulue et remplissant ladite pipette du liquide contenu dans la section isolée de la tubulure fermée 1.

Une fois la pipette 8 remplie, l'usager la déconnecte de la tubulure fermée 1 par un geste de torsion, tout écoulement de sang de la tubulure est évité grâce à la valve anti-retour de la membrane 6 qui se ferme.

Enfin l'usager peut déposer de fines gouttes du sang contenu dans la pipette 8 en comprimant celle-ci. Du fait de la finesse du conduit 7 de la pipette, les gouttes sont très fines et ne s'écoulent pas d'elles-mêmes hors de la pipette 8.

On peut donc prélever d'une tubulure fermée intégrée à la poche de transfusion sanguine, une faible quantité de sang en toute sécurité et en évitant toute contamination ou souillure.

Il est important de noter que la pipette 7, 8, du fait des manipulations nécessaires et obligatoires en laboratoire de la poche de transfusion doit:
1) être de petite taille, afin de n'engendrer aucune gêne lors de la manipulation, effectuée, au laboratoire, pour le conditionnement de la poche. Ainsi elle sera plus facilement maniable et intégrable lors de la compression dans le bac de la centrifugeuse et de la centrifugation de la poche.
2) La petite taille de la pipette devra permettre de prélever uniquement la quantité de sang nécessaire pour le contrôle pré transfusionnel 0,5 millilitre soit + ou - 8 gouttes. Deux gouttes nécessaires au contrôle pré transfusionnel et les autres gouttes prévues en cas de vérification du premier geste ou de perte accidentelle de sang par le manipulateur, lors du contrôle.
3) Etre fabriquée dans un matériau obligatoirement souple, de la même composition que la tubulure, ceci évitant tout éclatement ou cassure de la pipette lors de la compression de la poche pendant la centrifugation, pouvant provoquer la souillure par le sang de la centrifugeuse et être source de contamination.
4) Etre enchâssée dans une deuxième paroi de protection faisant partie intégrante de la tubulure, par soudure et assurant l'étanchéité totale du système, le sécurisant et évitant toute désolidarisation et ainsi toute forme de contamination lors de toutes manipulations de la poche pendant son remplissage ou son conditionnement au laboratoire.
5) Etre malgré sa petite taille d'une manutention aisée pour le manipulateur responsable du contrôle, et dans l'idéal être un renflement, de la tubulure, détachable, sécable uniquement par un mouvement de torsion exécuté volontairement, évitant ainsi le détachement accidentel de ladite pipette, et le risque de souillure.

L'innovation de ce perfectionnement du dispositif, permet de remédier au risque de cassure, d'éclatement, de désolidarisation accidentelle de la pipette, lors des différentes manipulations, compression, et centrifugation obligatoires.

En effet, par les améliorations apportées, le dispositif ainsi réalisé, devient de par sa petite taille, de par sa souplesse, de par sa protection supplémentaire, de par son ancrage résistant, plus maniable, plus malléable et plus sécurisé évitant au maximum tout risque de souillure et de contamination.

La détermination de la quantité de sang devient aussi un élément supplémentaire à la réalisation de l'invention.

## Revendications

1. Dispositif de prélèvement d'une petite quantité de sang contenu dans la tubulure fermée (1) d'une poche de transfusion sanguine, notamment pour réaliser un Contrôle Pré-transfusionnel Ultime (CPU), comprenant une pipette (7, 8) reliée à ladite tubulure fermée (1) **caractérisé par le fait qu'**il comporte une membrane à valve anti-reflux ou auto réparatrice intégrée dans la paroi de la tubulure fermée au point de liaison de celle-ci avec la pipette (7, 8); qu'il comporte encore une enveloppe protectrice (9), entourant la pipette (7, 8) moulée de façon étanche sur la tubulure fermée (1) autour de la pipette (8) et de son petit conduit (7); l'enveloppe de protection s'ouvrant par écartement de ses parois et restant sur la tubulure afin de permettre la préhension de la seule pipette; le tout étant agencé de manière à ce que la pipette puisse être séparée manuellement de la tubulure fermée (1) par un mouvement de torsion de la pipette (7, 8) par rapport à la tubulure fermée (1).

2. Dispositif selon la revendication 1, **caractérisé par le fait qu'**il est porté par un tronçon de la tubulure fermée (1) isolée tant de la poche de transfusion que du reste de la tubulure fermée (1).

3. Dispositif selon la revendication 1 ou la revendication 2, **caractérisé par le fait que** la pipette (7, 8) est relié à la tubulure fermée (1) par un petit conduit (7) dont la section est comprise entre 0,1 et 0,5 fois la section de la tubulure fermée (1).

4. Dispositif selon l'une des revendications précédentes, **caractérisé par le fait que** le volume de la pipette (8) est de 1,0 ml. soit l'équivalent de 20 gouttes.

5. Dispositif selon l'une des revendications précédentes, **caractérisé par le fait que** la tubulure fermée (1), la pipette (7, 8) et l'enveloppe protectrice (9) sont formées d'un même matériau.

6. Dispositif selon l'une des revendications précédentes, **caractérisé par le fait que** l'enveloppe protectrice (9) comprend deux feuilles soudées ou venues d'une pièce de fabrication par une de leurs extrémités avec la tubulure fermée (1) et comportant des formations d'emboîtement permettant en les rapprochant de former une enceinte souple et étanche autour de la pipette (7, 8).

7. Poche de transfusion comportant une tubulure fermée (1), **caractérisée par le fait que** cette tubulure fermée (1) comporte un dispositif de prélèvement selon l'une des revendications 1 à 6.
